Europäisches Patentamt

**European Patent Office**

Office européen des brevets

(11) Publication number: **0 211 114**
**A1**

# (12) EUROPEAN PATENT APPLICATION

(21) Application number: **85305406.2**

(51) Int. Cl.⁴ **A61B 17/12**

(22) Date of filing: **30.07.85**

(43) Date of publication of application:
**25.02.87 Bulletin 87/09**

(84) Designated Contracting States:
**AT BE CH DE FR GB IT LI LU NL SE**

(71) Applicant: **AMERICAN HOSPITAL SUPPLY CORPORATION**
**One American Plaza**
**Evanston, IL 60201(US)**

(72) Inventor: **Blake, Joseph W. III**
**88, Main street**
**New Canaan Connecticut 06840(US)**

(74) Representative: **Johnson, Terence Leslie et al**
**Edward Evans & Co. Chancery House 53-64**
**Chancery Lane**
**London WC2A 1SD(GB)**

(54) Ligator device.

(57) A ligator device has handles (1,2) which are linked to a center slide (13) to make the slide (13) move rearwardly when the handles are squeezed together. A pair of jaws (14,15) surround the front of the slide (13) top and bottom and are connected to it so as to open when the handles are released and close when the handles are squeezed. The forward ends of the jaws carry anvils (11,12) which, when closed, squeeze shut ligating clips supplied from a magazine.

FIG. 2

EP 0 211 114 A1

# LIGATOR DEVICE

The present invention is concerned with a ligator device, i.e., a forceps-like device which dispenses ligating clips and applies them to blood vessels and other small fluid ducts to close the same.

It is a general object to the present invention to provide a simpler, less complicated ligating device.

A more particular object of the invention is to provide an improved ligating device which is simple and uncomplicated in its construction, but is nevertheless wholly reliable in operation.

Still another object of the invention is to provide such an improved ligating device which can readily be made of disposable materials, so that it can be discarded when it has served its use.

In keeping with these objects, and with still others which will become apparent as the description proceeds, one aspect of the invention resides in a ligating device which, briefly stated, may comprise a ligator device comprising a pair of handles, a longitudinally movable center slide linked to the handles so as to move rearward when the handles are squeezed together, a pair of jaws each slidably connected to a front portion of the center slide and each having a front end provided with an anvil, and means connecting the jaws with the center slide for the anvils to move together when the handles are squeezed and for the anvils to move apart when the handles are released.

The invention will hereafter be described with reference to an exemplary embodiment. However, it should be understood that this is for purposes of explanation only and is not to be considered limiting the invention in any sense. A ligator device embodying the invention is described, by way of example, with reference to the accompanying drawings.

FIG. 1 is a top plan view of a ligator device embodying the present invention;

FIG. 2 is a diagrammatic top plan view, partly broken away and with still other parts removed, of the same ligator device as in FIG. 1;

FIG. 3 is a section taken on line III-III of FIG. 2;

FIG. 4 is an exploded view, illustrating certain components of the device in FIGS. 1-3; and

FIG. 5 is an enlarged plan view, illustrating a detail of the device.

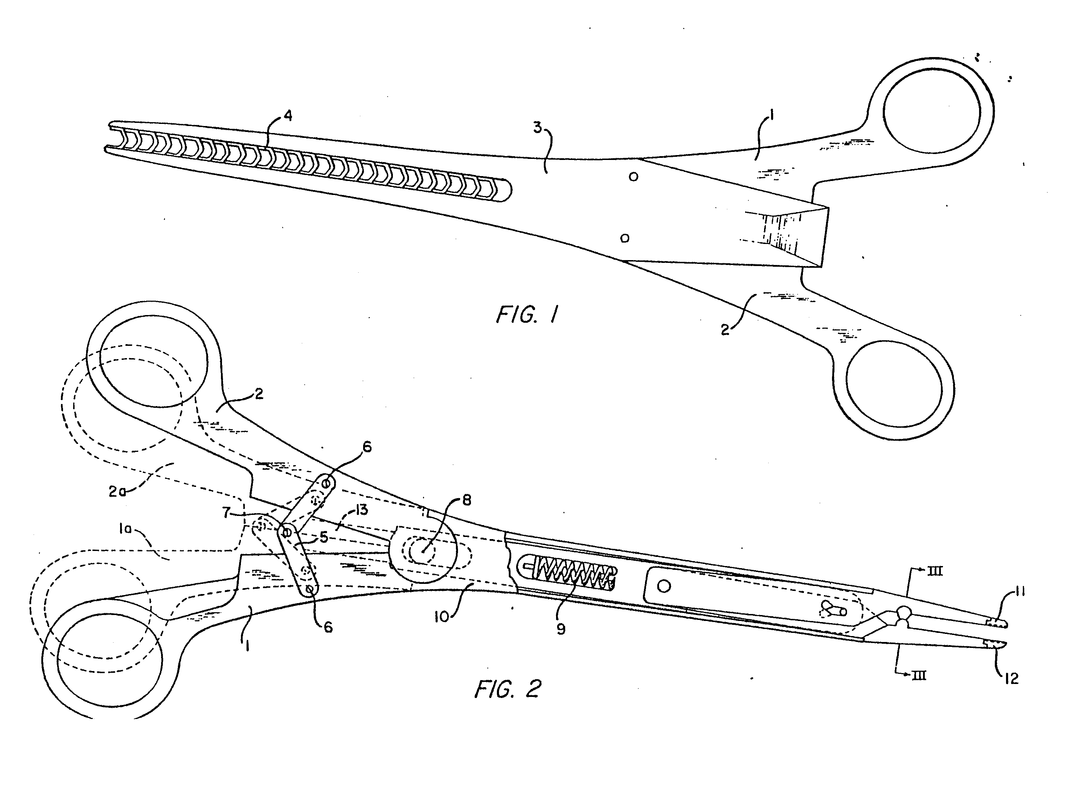

FIG. 1 shows the device in a top plan view to provide in effect an overview and a general understanding. A pair of scissor-like handles 1, 2 are linked with one another (not shown) to operate a pair of jaws (not shown). Seated on top of the device is an elongated cartridge 3 which contains a series of ligating clips 4. These are seriatim dispensed to the jaws (every time the two handles are squeezed together and then released) which then apply them to the blood vessel or the like.

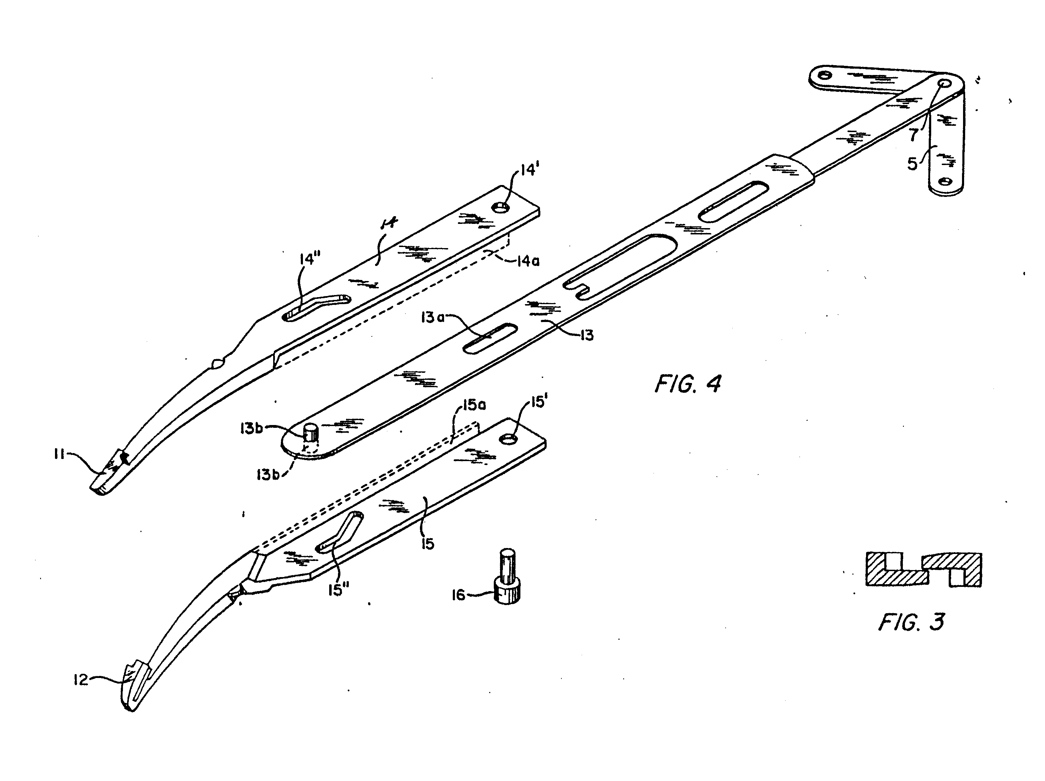

The device is shown in more detail in FIGS. 2 and 3. Moved (i.e., squeezed-together or clip-setting) positions of the handles are indicated at 1a and 2a. The handles 1, 2 are connected by a two-part triangular link 5 whose ends are pivoted to the handles at 6 and whose other ends are pivoted to one another at 7. The handles 1, 2 themselves are pivoted to one another at 8. A spring 9 is secured at one end to a stud of the (broken-away) housing 10 and at the other end to a center slide 13 to retract the same. At the front end, the device has anvils 11, 12 which squeeze the ligating clips to closed position. The magazine is omitted in this illustration.

As the section III-III (FIG. 3) shows, the cross-section of the jaws is so chosen that in the closed - (clip-setting) position the jaws stabilize one another so that there can be no "wobbling" or similar movement. Most parts of the device, except for the spring 9, the center slide 13 and the anvils 11, 12, can be made of synthetic plastic material.

FIG. 4 is an exploded view, showing the jaws by themselves. Reference numeral 13 identifies the center slide (see also FIG. 2) to the rear of which the link 5 is pivoted at 7. It is bracketed top and bottom by a pair of jaws 14 and 15. Jaw 14 has a depending wall 14a at one side, jaw 15a a similar but upstanding wall 15a (both of these could be omitted) at the other side, so that between themselves they securely hold the center slide 13 against lateral break-out movement. The rear ends of jaws 14, 15 have holes 14', 15' and the center slide 13 is provided towards the rear with a longitudinally extending cut-out 13a. A pin 16 extends through cut-out 13a and holes 14', 15' to hold the pieces together.

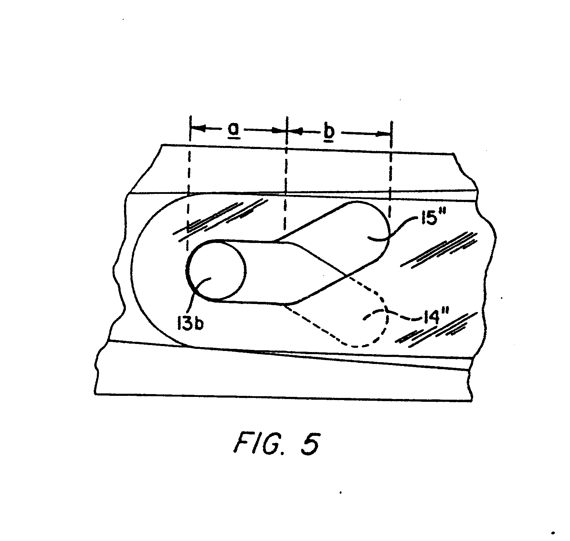

About midway, the jaws 14, 15 are formed with angled slots 14", 15" into which a pin 13b of center slide 13 extends. This is shown in more detail in FIG. 5 from where it will also be understood that the slot region a provides the necessary delay for retraction of the clips upstream of the one in the jaws whereas the region b provides the camming action for closing the jaws and setting the clip by the anvils 11, 12.

The invention has hereinbefore been described with reference to an exemplary embodiment. However, various modifications and changes will offer themselves to those skilled in the art and these are intended to be encompassed within the scope of the appended claims.

## Claims

1. A ligator device, characterised by:

a pair of handles (1,2);

by a longitudinally movable center slide (13) linked to said handles (1,2) so as to move rearward when the handles are squeezed together;

by a pair of jaws (14,15) each slidably connected to a front portion of said center slide (13) and each having a front end provided with an anvil (4,12); and

by means (5,8,9) connecting said jaws (14,15) to said center slide for said anvils to move together when said handles are squeezed, and for said anvils to move apart when the handles are released.

2. A ligator device according to claim 1, characterised in that at least said anvils (11,12) are of metal and in that at least some other mentioned parts are of synthetic plastic material.

3. A ligator device according claim 1 or claim 2, characterised by a cartridge (3) loaded with a supply of ligating clips (4).

4. A ligator device according to claim 3, characterised by means for retracting all except the respectively leading clip (4) located between the anvils (11,12), in direction rearwardly away from said anvils, prior to movement of said anvils together.

5. A ligator device according to claim 4, characterised in that said retracting means comprises a slot (14") in one of said jaws (14) and angled crosswise thereof in one direction, and a slot (15") in the other jaw (15) and angled crosswise thereof in the opposite direction, and in that there is a pin (13b) integral with said front portion of said center slide (13) and extending through said slots (14" and 15").

FIG. I

FIG. 2

0 211 114

FIG. 4

FIG. 3

0 211 114

FIG. 5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | GB-A-2 056 898 (J.W. BLAKE) * Page 1, line 110 - page 2, line 28; figures 1,3,4,11A,11B,11C * | 1-4 | A 61 B 17/12 |
| X | US-A-2 194 748 (M.A. GLASER) * Claims 1,2; figures 1,2,7 * | 1,3,4 | |
| A | US-A-4 452 357 (KLIEMAN et al.) * Column 3, lines 29-50; figures 1,3,4,7 * | 1,3 | |
| A | GB-A-2 073 022 (D.T. GREEN) | | |

-----

|  | TECHNICAL FIELDS SEARCHED (Int. Cl.4) |
|---|---|
|  | A 61 B |

The present search report has been drawn up for all claims

| Place of search | Date of completion of the search | Examiner |
|---|---|---|
| THE HAGUE | 06-03-1986 | MARTINOZZI G.M.E. |